# FASCICULE DE BREVET EUROPEEN

(11) **EP 1 372 683 B1**
(45) Date de publication et mention de la délivrance du brevet: **08.03.2006**
(21) Numéro de dépôt: 02722392.4
(22) Date de dépôt: 03.04.2002
(51) Int. Cl.: A61K 36/9062, A61P 17/00

(54) **UTILISATION D'UN EXTRAIT DE RHIZOMES D'ALPINIA OFFICINARUM**
VERWENDUNG EINES ALPINIA OFFICINARUM WURZELSTÖCKE EXTRAKTES
USE OF AN EXTRACT OF ALPINIA OFFICINARUM RHIZOMES

(30) Priorité: 05.04.2001 FR 0104634
(43) Date de publication de la demande: 02.01.2004
(73) Titulaire: Platzgummer, Emile, 06800 Cagnes sur Mer (FR)
(72) Inventeur: PLATZGUMMER, Emile, 06800 Cagnes sur Mer (FR)
(74) Mandataire: Vuillermoz, Bruno
(86) Numéro de dépôt international: PCT/FR2002/001148
(87) Numéro de publication internationale: WO 2002/080950

(56) Documents cités:
- PATENT ABSTRACTS OF JAPAN vol. 011, no. 315 (C-451), 14 octobre 1987 (1987-10-14) & JP 62 099325 A (SHISEIDO CO LTD), 8 mai 1987 (1987-05-08)

## Description

L'invention concerne une nouvelle utilisation d'un extrait de rhizomes d'Alpinia Officinarum, en particulier pour le traitement du vitiligo.

Le vitiligo est une infection bien connue caractérisée par un trouble de la pigmentation de la peau se manifestant par la présence de plaques décolorées d'un blanc mat à contours précis, entourées d'une zone où la peau est plus pigmentée que normalement.

De nombreuses compositions à usage externe notamment, ont été proposées pour le traitement de cette infection.

Le document CN 1 192 368 décrit par exemple un mélange d'herbes traditionnelles chinoises, comprenant notamment une plante dénommée Heiguzi, susceptibles d'être appliqué directement sur les tâches et supposé faire disparaître leur intensité en un temps très court.

Le document CN 1 181 970 décrit quant à lui une préparation à base de plantes traditionnelles chinoises comprenant notamment du Fructus Psoralae, et du Radix Sophorae Flavescentis. Ce mélange est obtenu par trituration, mélange et filtration des plantes de manière à obtenir une solution liquide. De même que précédemment, le produit obtenu est une solution à usage externe appliquée localement.

D'autres documents tels que les documents CN 1 170 602 et CN 1128 152 décrivent également des compositions à base de plantes toujours mises en oeuvre en usage externe par application locale.

Le document JP 62 099325 décrit l'utilisation de rhizomes d'Alpinia Officinarum pour le traitement des brûlures après exposition au soleil, le traitement des crevasses ou gerçures, de l'acné et enfin des irritations après rasage.

Le Demandeur a constaté que de manière tout-à-fait surprenante la plante dénommée Galanga, connue également sous la dénomination Alpinia Officinarum et notamment son rhizome, présentait des propriétés thérapeutiques, en particulier vis-à-vis du vitiligo.

Cette application n'est ni décrite, ni suggérée par le document JP 62 099325 qui ne concerne que des pathologies n'ayant aucun point commun avec le vitiligo correspondant, comme déjà dit, à un trouble de la pigmentation de la peau.

L'invention concerne donc l'utilisation d'un extrait de rhizome d'Alpinia Officinarum pour la fabrication d'un médicament destiné au traitement du vitiligo.

La Galanga est une plante bien connue provenant de Chine faisant partie de la famille des zingibéracées. Le rhizome amylacé de cette plante se présente en fragments cylindriques souvent ramifiés, de 5 à 10 cm de longueur et de 13 à 20 mm de diamètre, à surface brun rougeâtre marquée d'anneaux circulaires frangés, représentant les restes des écailles foliacées. La coupe transversale montre une écorce épaisse de couleur cannelle, renfermant de nombreux faisceaux follières. Le cylindre central est en outre parcouru par des faisceaux plus nombreux très rapprochés les uns des autres. L'odeur du Galanga est aromatique et épicée, la saveur âcre est brûlante.

Le rhizome est utilisé de manière connue comme excitant, et stomachique comme condiment aromatique. Dans ses applications, le rhizome est administré à raison de 1 à 3 g par jour.

Les terpenoïdes présents dans le rhizome de l'Alpinia Officinarum ont également été décrits dans le document WO 99/53935 pour leur utilisation dans la prévention des réactions allergiques. De telles compositions sont obtenues par extraction du type macération, percolation ou extraction super critique du rhizome en solvant organique.

L'extrait de rhizome de l'invention est obtenu par les procédés d'extraction connus de l'homme du métier, tels que macération, percolation, etc..., à chaud ou à froid.

Dans un mode de réalisation avantageux, l'extrait est obtenu par un procédé selon lequel :
- on retire tout d'abord l'écorce des rhizomes ;
- on réduit ensuite la taille desdits rhizomes ;
- on laisse macérer les fragments de rhizomes dans un solvant ;
- on filtre enfin la solution pour en récupérer la partie liquide.

Pour résoudre le problème de la réduction de taille des rhizomes, lesdits rhizomes sont coupés en rondelles, puis éventuellement ensuite mixés.

Par ailleurs, l'extraction est effectuée comme déjà dit par macération, avantageusement à froid.

Pour disposer d'une composition utilisable en application locale, le solvant est un solvant pharmaceutiquement acceptable choisi dans le groupe comprenant le vinaigre de cidre ou encore l'huile d'olive, le choix de l'un ou l'autre de ces solvants dépendant de la réactivité de l'épiderme du patient.

Pour obtenir une extraction maximale, la macération à froid est comprise entre 2 et 20 jours, avantageusement 10 jours.

L'extrait peut être utilisé tel quel ou sous forme de gel, pommade, lotion, crème, le produit obtenu à l'issue de l'étape de macération étant incorporé dans un excipient adéquat, dont le choix est à la portée de l'homme du métier.

Le Demandeur a par ailleurs constaté que de façon tout-à-fait surprenante, l'extrait de rhizomes de Galanga était également efficace pour lutter contre l'apparition des tâches de senescence.

Dès lors, l'invention concerne également l'utilisation de l'extrait précédemment décrit pour la fabrication d'une composition cosmétique destinée à atténuer les tâches de senescence.

Dans toutes ces applications, l'extrait utilisé en application locale est administré à raison de une à deux applications par jour pendant une période comprise entre 15 et 90 jours.

L'invention et les avantages qui en découlent ressortiront mieux des exemples de réalisation suivants.

### Préparation de la composition

- on retire soigneusement l'écorce de 200 g de rhizomes d'Alpinia Officinarum ;
- on coupe ensuite les rhizomes en fines rondelles ;
- on laisse enfin macérer lesdites rondelles dans 100 ml de vinaigre de cidre pendant 10 jours à froid ;
- on filtre et on récupère la partie liquide.

### Traitement du vitiligo

On applique localement la composition ci-dessus préparée une fois par jour pendant 30 jours.

Après 30 jours, on observe la complète disparition du vitiligo.

L'invention et les avantages qui en découlent ressortent bien de la description. On note en particulier les propriétés intéressantes de la composition décrite, notamment dans le traitement du vitiligo et celui des tâches de senescence.

## Revendications

1. Utilisation d'un extrait de rhizomes d'Alpinia Officinarum pour la fabrication d'un médicament destiné au traitement du vitiligo.

2. Utilisation d'un extrait de rhizomes d'Alpinia Officinarum selon la revendication 1, **caractérisé en ce que** l'extrait est obtenu par un procédé selon lequel :
- on retire tout d'abord l'écorce des rhizomes ;
- on réduit ensuite la taille desdits rhizomes ;
- on laisse macérer les fragments de rhizomes dans un solvant ;
- on filtre enfin la solution pour en récupérer la partie liquide.

3. Utilisation selon la revendication 2, **caractérisée en ce que** le solvant est un solvant pharmaceutiquement acceptable choisi dans le groupe comprenant le vinaigre de cidre et l'huile d'olive.

4. Utilisation selon la revendication 1, **caractérisée en ce que** l'extrait est incorporé dans un gel, crème, lotion ou pommade.

5. Utilisation selon la revendication 1 en application locale à raison de une à deux applications par jour pendant une période comprise entre 15 et 90 jours.

## Claims

1. Use of an extract of alpinia officinarum rhizomes to produce a medication intended for treating vitiligo.

2. Use of an extract of alpinia officinarum rhizomes as claimed in claim 1, **characterised in that** the extract is obtained by a method whereby:
- the outer skin is first removed from the rhizomes;
- the size of said rhizomes is then reduced;
- the pieces of rhizome are then left to macerate in a solvent;
- the solution is then filtered in order to collect the liquid.

3. Use as claimed in claim 2, **characterised in that** the solvent is a pharmaceutically acceptable solvent selected from the group comprising cider vinegar and olive oil.

4. Use as claimed in claim 1, **characterised in that** the extract is incorporated in a gel, cream, lotion or ointment.

5. Use as claimed in claim 1, applied topically one to two times a day over a period of 15 to 90 days.

## Patentansprüche

1. Verwendung eines Extrakts von Alpina officinarum-Wurzelstöcken zur Herstellung eines Medikaments, das zur Behandlung der Weißfleckenkrankheit (Vitiligo) bestimmt ist.

2. Verwendung eines Extrakts von Alpina officinarum-Wurzelstöcken nach Anspruch 1, **dadurch gekennzeichnet, dass** der Extrakt durch ein Verfahren erhalten wird, nach dem
- zuerst die Rinde der Wurzelstöcke entfernt wird,
- die Wurzelstöcke dann zerkleinert werden,
- die Fragmente der Wurzelstöcke in einem Lösungsmittel eingeweicht werden,
- schließlich die Lösung filtriert wird, um den flüssigen Anteil zu gewinnen.

3. Verwendung nach Anspruch 2, **dadurch gekennzeichnet, dass** das Lösungsmittel ein pharmazeutisch annehmbares Lösungsmittel ist, das aus der Gruppe ausgewählt ist, die aus Apfelweinessig und Olivenöl besteht.

4. Verwendung nach Anspruch 1, **dadurch gekennzeichnet, dass** der Extrakt in ein Gel, eine Creme, Lotion oder Salbe eingebracht wird.

5. Verwendung nach Anspruch 1 zur lokalen Anwendung mit einer oder zwei Anwendungen pro Tag während einer Zeitspanne zwischen 15 und 90 Tagen.
